(19) Europäisches Patentamt
European Patent Office
Office européen des brevets

(11) **EP 3 916 603 A1**

(12) **EUROPEAN PATENT APPLICATION**

(43) Date of publication:
**01.12.2021 Bulletin 2021/48**

(51) Int Cl.:
*G06F 21/62* (2013.01)    *G06Q 30/02* (2012.01)

(21) Application number: **20382454.5**

(22) Date of filing: **28.05.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koa Health B.V.**
**1097 JV, Amsterdam (NL)**

(72) Inventors:
• OMAÑA, Jesús
  **28050 MADRID (ES)**
• SIMAS, Tiago
  **28050 MADRID (ES)**

(74) Representative: **Herrero & Asociados, S.L.**
**Cedaceros, 1**
**28014 Madrid (ES)**

(54) **METHOD AND SYSTEM FOR EFFICIENT USERS' DATA EXTRACTION AND USERS' WELLBEING IMPROVEMENT BASED ON USERS' ELECTRONIC DEVICES INTERACTIONS**

(57)    A method and system for efficient repertory grid data extraction and well-being improvement recommendations. This method and system propose a novel way to collect users' data from user application interactions (in electronic devices) that enables to extract repertory grids or personal constructs from one or more users, in such a way that the users do not explicitly has to fill the repertory grid data. This structured way of collecting data allows to extract Individual Personal Constructs in a massively scalable fashion with minimum resources. Additionally, the extracted data may be recurrently input into a novel recommender system or engine which would result in higher engagement efficacy for the user, helping the individual on his/her decision-making process.

FIG. 1

**Description**

**Field of the invention**

The present invention has its application within the telecommunications sector, more specifically, relates to the deployment of tools using electronic devices and communication electronic devices (e.g., mobile user terminals such as smartphones or tablets or for wearable electronic devices, computers, etc.) which measure and send user's parameters and/or interact with the user, to estimate and improve his/her wellbeing (for example, alleviating stress). More particularly, the present invention relates to a method and electronic system for an efficient extraction of user's well-being data and recommendations (similar to what personal construct theory does face-to-face with a psychiatrist), which allows to derive accurate recommendations to improve the user's well-being.

**Background of the invention**

Since 2008, the leading cause of death in the United States has become personal choices/behaviours, and the same trend is seen in many countries worldwide. In order to be able to change behaviours that damage the health of an individual, it has to be understood first the personal constructs of such individuals on certain topics - in other words, it has to be understood how an individual reacts to certain events/circumstances -. In this regard, within the personal construct theory exists repertory grids, which is an interview technique (developed by George Kelly) that has proven to be successful on extracting from the individual the ways (s)he extracts meaning from the observations/events that occur to him/her or around him/her.

The repertory grid is an interviewing technique which uses non-parametric factor analysis to determine a measure of personality. In other words, the repertory grid is a technique for identifying the ways that a person construes (interprets or gives meaning to) his or her experience. It provides information from which inferences about personality can be made, but it is not a personality test in the conventional sense.

The repertory grids are based on the personal construct theory developed by George Kelly in which he describes humans as "incipient scientists" who creates hypothesis in order to understand and interpret events, in this context, the repertory grid is a technique designed to explore the structure and content of these hypotheses. Mathematical techniques to extract meaning from repertory grids have been developed because an interesting feature of the repertory grid technique is that the results could be analysed mathematically. In this regard some approaches have been proposed, as for example shown in: Faccio, E., Castiglioni, M., & Bell, R. C. (2012). Extracting information from repertory grid data: New perspectives on clinical and assessment practice. TPM - Testing, Psychometrics, Methodology in Applied Psychology, 19(3), 177-196.

Repertory grids techniques have been applied in different domains, for example in clinical, subclinical and marketing settings, among others. For instance, repertory grid techniques had been used for assessing work-related stress or for assessing whether a given treatment would be effective.

Repertory grid interviews are usually performed between a psychiatrist and a patient (face to face) and typically it takes around one hour from start to finish, thus making it not feasible to be implemented at scale. Generally speaking, the repertory grids techniques as applied until now, have some common requirements/characteristics that make them not efficient to be applied fast, resource savings and at large scale (applicable to a huge number of users). Said requirements are:

- Location: The interview must be performed in an office (or any other predefined location) - i.e. it cannot be taken in whichever place the user wants.
- Scale: It requires a psychiatrist to perform the interview face-to-face with the user.
- Time: Typical repertory grid interviews last between 45min to more than one hour, the reason is mainly that the psychiatrist will aim to explore as much as possible the answers from the users given that it is not convenient to bring the user to a predefined location (e.g. the psychiatrist office) multiple times.
- No-anonymity: The individual (the user or, in other words, the person to which the repertory grid interview is made) must feel comfortable telling his/her psychiatrist his personal views on a given topic -- thus, feelings of judgment and shame might not allow the user to express him/herself as honest as (s)he would like to.

Therefore, a need exists for a method and system for applying repertory grids to users in an accurate, personalised and resources saving way and allowing to derive accurate recommendations for behaviour modification to improve the user's wellbeing (avoiding undesired behaviours in the future).

**Summary of the invention**

The problems found in prior art techniques are generally solved or circumvented, and technical advantages are generally achieved, by the disclosed embodiments which provide a method and system for efficient application of repertory grid techniques and well-being improvement recommendations. The proposed method and system propose a novel way to collect users' data from users application interactions (in electronic devices) that enables to extract repertory grids (also called repertory grids data) or personal constructs from one or more users, in such a way that the users do not explicitly has to fill the repertory grid data. Hence, it can be said that the repertory grids are extracted in a passive way (that is, without requiring that a user actively provides the repertory grids, for example, by answering to specific questions), so the extracted repertory grids can be denominated Passive Digital Repertory Grids (PDRG).

Then, PDRG is a structured way to collect data that allows the inference of users' Personal Constructs (a way to psychologically characterize an individual). This allows to assess a user's psychological state, which can be modelled and fed to a recommender engine towards the goal to help improving the individual's wellbeing.

It is proposed to leverage the design of some electronic device applications (for example, applications aimed to reduce stress or any other type of application), in order to extract passive digital repertory grids (PDRGs) such that individual (user) personal constructs can be understood and such that the user does not have to explicitly provide the repertory grids data. That is, the PDRGs would be acquired based on how the user interacts with an electronic device application (e.g. a smartphone application), having the advantage of constantly and passively (from the user's point of view) acquiring the repertory grids for the user, in a similar way as a specialist would get with a personal interview. The repertory grid technique has so far not been integrated into any (smartphone) application, in such a way that the user does not explicitly fill the repertory grid.

One of the advantages of the proposed solution (when compared with the problems of the current repertory grids techniques as previously stated) is that it will allow to extract Individual Personal Constructs in a massively scalable fashion with minimum resources. Therefore, it will allow to create group characterization and comparison between many users (individuals), which is only possible when reaching a large scale of users.

Additionally, PDRGs could be recurrently input into a novel recommender system or engine (RecSys) which would result in higher engagement efficacy for the user, helping the individual on his/her decision-making process. That is, in a novel approach, the present solution proposes to feed the obtained personal constructs of users as input for a recommender system. In one embodiment, the recommender system uses state vectors and diffusion metrics in order to propose activities that will be easier (i.e. proposes activities that create a smooth transition from the current to the desired state the user) for the user to engage (measured, for instance, through click rate) and obtain better results (i.e. increase well-being or reduce stress).

In a first aspect, it is proposed a computer implemented method for efficient extraction of personal users' data and improvement of the wellbeing state of users, the method comprising the following step performed by one or more electronic processors of an electronic system:

a) Receiving and storing in a database, data related to one or more users, where data related to a user is acquired through interactions of the user with an application running in an electronic device of said user (via an user interface of the electronic device);

b) Generating a graphical model for at least one user (from the one or more users whose data related to said users has been stored in the database) by processing a set of data related to said user, obtained from the database (data acquired through interactions of the user with an application running in an electronic device of said user), corresponding to a certain period of time; where nodes of the graphical model correspond to the different values of the set of data;

c) For each user for which a graphical model has been generated, determining a recommended range of values for parameters or activities related to the user (which usually should result on an improvement of the wellbeing score of the user) based at least on the generated graphical model for the user, using state vectors and diffusion metrics obtained from the graphical model generated for the user.

The state vector may be perceived from the application running in the electronic device capturing data along time e.g. in the form (t, current state variable, future state variable), for example by the user stating how she/he feels and how she/he wants to feel in the future. The application used to acquire the set of data related to the user (through user interactions with said application) may be many software applications, as for example, a user's well-being application such as REMIX or any other user's well-being application.

The database may be a database external to the electronic devices of the one or more users (that is, a database stored in an electronic device external to the user's electronic devices), and the electronic devices of the one or more users send the acquired data to the external device storing the database (for example a remote server), through a communications network.

In an alternative embodiment, as the graphical model is generated per user, the technique proposed has the ability to run without having to store data in a server, instead, using the data stored in the user electronic device.

The one or more electronic processors performing the method (and the database) may be located in an electronic device of one of the users. Or, the one or more processors (and the database) may be located in one or more remote servers (external to the user's electronic devices) and the one or more users' electronic devices acquiring the data related to the users send, through a communications network, the acquired data to the remote server(s).

The electronic device of each user may be any electronic device, for example: a laptop, a tablet, a personal computer, a portable computer, a mobile phone, a smartphone or a smartwatch.

In an embodiment, step b) further includes updating the graphical model based on a new set of data acquired through the application running in the electronic device of the user, corresponding to a different later period of time (later than the certain period of time); and/or updating the generated graphical model for an user every time new data for said user, acquired through user interactions with the application running in the user's electronic device, is received and stored in the database, after the certain period of time.

In an embodiment, the set of data related to a user is a temporal ordered list of pairs of values of personal construct, X, and opposed personal construct, Y, of the user corresponding to the certain period of time and the graphical model for an user includes a graph, Bipartite Graph, based on the frequency that a specific pair of values of personal constructs and opposite personal construct (X,Y) appear in the list (the weights of the edges in the graphical model are, for example, given by the frequency a specific pair of personal constructs and opposite personal construct (X,Y) appear in the list). Said graphical model for the user may comprise an extended graph which includes the Bipartite Graph, the projection of the Bipartite Graph to the personal construct nodes X and the projection of the Bipartite Graph to the opposed personal construct nodes Y, where said projections are built using a similarity measure (for example, the Fuzzy Jaccard Similarity measure). Said extended graph may also include the transpose of the Bipartite Graph.

Optionally, to build said extended graph, the scale effect between the Bipartite Graph and the projections is reduced (for example, using a z-score technique).

The personal construct and the opposed personal construct may be a current state and a desired state respectively of the user.

In an embodiment, step c) comprises:

c1) obtaining and initial state and a target wellbeing goal for an user;
c2) based on the graphical model generated for said user and on the initial state and the target goal, apply n-diffusion closure distance to determine the shortest diffusion path between the initial state ant the target goal;
c3) based on said determined shortest diffusion path, determining a recommended range of values for parameters or activities related to the user for achieving the target goal from the initial state of the user.

The determined recommendation for a user (recommended range of values for parameters or activities related to the user) may be presented to the user through an user interface of the electronic device of the user.

In a second aspect, it is proposed systems to perform the above stated proposed methods. For example, in this second aspect it is proposed an electronic system for efficient extraction of personal users' data and improvement of the wellbeing state of users, the system comprising:

- Means for receiving and storing in a database, data related to one or more users, where data related to an user is acquired through interactions of the user with an application running in an electronic device of said user;
- at least one processor configured to:

  - Obtain from the database a set of data (related to at least one user) corresponding to a certain period of time;

  - Generate a graphical model for the at least one user by processing the set of data of related to said user corresponding to the certain period of time; where nodes of the graphical model correspond to different values of the set of data;

  - For each user for which a graphical model has been generated, determining a recommended range of values for parameters or activities related to the user based at least on the generated graphical model for the user, using state vectors and diffusion metrics obtained from the graphical model generated for the user.

A last aspect of the invention refers to a computer program product comprising computer program code adapted to perform the method of the invention, when said program code is executed on a computer, a digital signal processor, a field-programmable gate array, an application-specific integrated circuit, a micro-processor, a micro-controller, or any other form of programmable hardware. A non-transitory digital data storage medium is also provided for storing a computer program which comprises instructions causing a computer executing the program to perform the above-described method.

**Description of the drawings**

For the purpose of aiding the understanding of the characteristics of the invention, according to a preferred practical embodiment thereof and in order to complement this description, the following figures are attached as an integral part thereof, having an illustrative and non-limiting character:

Figure 1 shows an schematic example on how a mobile application (REM!X) can be used for repertory grids extraction.

Figure 2 shows a schematic example of how the extended graph is built from the bi-partite graph (BG), according to an embodiment of the present invention.

**Detailed description of embodiments of the invention**

The matters defined in this detailed description are provided to assist in a comprehensive understanding of the invention. Accordingly, those of ordinary skill in the art will recognise that variation changes and modifications of the embodiments described herein can be made without departing from the scope of the invention. Also, description of well-known functions and elements are omitted for clarity and conciseness.

Note that in this text, the term "comprises" and its derivations (such as "comprising", etc.) should not be understood in an excluding sense, that is, these terms should not be interpreted as excluding the possibility that what is described and defined may include further elements, steps, etc.

Of course, the embodiments of the invention can be implemented in a variety of architectural platforms, operating and server systems, devices, systems, or applications. Any particular architectural layout or implementation presented herein is provided for purposes of illustration and comprehension only and is not intended to limit aspects of the invention.

The proposed embodiments provide a method and system for efficiently extracting personal construct of the users and providing well-being improvement recommendations (e.g. by feeding the extracted user's constructs to a recommender engine) for changing user's damaging behaviors.

In order to understand and change user's behaviour, it is fundamental to get a better understanding of how the human mind works. The human brain and mind are multiscale adaptive self-organizing complex systems. Brain and mind can be modelled as a set of complex associations or complex networks. By hypothesis, behaviour relies on an associative set of memories that are built-up through the individual interaction with the environment and the replaying of the associative memory network. Mind can be seen as an adaptive self-organizing multiscale network of memories distributed in a manifold that folds space/time perception of the user.

The proposed embodiment approaches the substrate acquisition to future exploit the human behaviour dynamics in space/time. In that regard, the repertory grids approach is used.

Specifically, a grid uses to consists of four parts or type of components:

A topic: it is about some part of the person's experience.
A set of elements (1... k) which are examples or instances of the topic. They can be for example some activities to reach a particular state related to the topic, some objects related to the topic, etc.
A set of constructs (1.... n). They can be for example current and desired states (related to the topic) of the user or generally speaking basic terms that the client uses to make sense of the elements. They use to be expressed as a contrast.
A set of ratings (of elements on constructs). Feedback form the user about the elements (for example about the recommended activity). For example, each element could be positioned between the two extremes of the construct using a rating scale system and this is done repeatedly for all the constructs that apply.

An example of a repertory grid about a topic, in short, is the relation between elements and constructs. For the topic "addiction", examples of elements may be: To consume Heroin, Cocaine, Alcohol, etc. which would be rated by its constructs and opposite construct, such as: (feel the buzz, not feel the buzz), (feel sexy, not feel sexy), (makes me sick, makes me healthy), (feel happy, feel sad), etc.

In general, the Repertory Grids technique currently consists of personal interviews about a given topic with the strategy of first priming or eliciting the subject interviewed with a set of initial questions that follows with the free elicitation by the subject that defines the set of elements and constructs and its relative association value. Repertory Grids are snapshots of this memory multi-scale adaptive self-organized associative complex system of an individual in space/time. That is, the interview refers to how the subject in question positions or values a set of elements relative to each other at the space/time of the interview. Most decision making varies in space/time i.e. depends on the context and value gain or cost reduction of the decision to be taken towards the goal.

However, traditional way of acquiring repertory grids lacks spatial and temporal resolution. As a solution to this problem, it is proposed hereby Passive Digital Repertory Grids (PDRG). PDRG differentiates from traditional repertory grids because in PDRG repertory grids over several topics is continuously obtained over space/time (through an electronic device application). A limitation is that users might not be willing to constantly answer questionnaires (so they cannot be continuous being interviewed); in order to avoid that, in the proposed solution several repertory grids (i.e. elements, constructs and ratings) are extracted based on interactions of the users with electronic device application (e.g. mobile applications).

In an embodiment, said applications are applications related to increase well-being (such as REM!X). In this specific app the user responds about his current (emotional/wellbeing) state and the (emotional/wellbeing) state he/she wants to achieve (which works as a dual construct) and the recommended activity works as the elements of the repertory grid. The rating of the recommended activity, values (rates) the elements regarding the construct. Over time, the app acquires the repertory grid of the subject in study with a good spatial and temporal resolution. Moreover, this type of approach also extends to a massive cohort of users.

The user's electronic devices running applications used to extract the repertory grids may be any one of the following: a laptop, a tablet, a personal computer, a portable computer, a mobile phone, a smartphone, a smartwatch ... or any other electronic device which the user can interact with and where an application can be run.

The invention does not depend on a particular type of electronic device or application. That is, many types of applications can be used, as long as the application is able to capture the repertory grids (PDRG) data and, preferably, store in a consistent (remote or local) database.

That is, the data captured over time for a specific user by the electronic device application is stored in a database. This database may be internal to one of the users' electronic devices or may be external to the users' electronic devices (for example, in a remote server). The electronic device should have a communication interface which allows the electronic device to communicate the acquired data to the database (directly or through another electronic device) over a communications network. The communications network may be a wireless communications network, a mobile communications network as GSM 2G, GPRS, UMTS, 3G, 4G or LTE, LAN or W-LAN or any other type of wired or wireless communication networks. For simplicity, it is supposed that all the captured data is stored in a single database but generally speaking the data can be stored in one or more databases in the same or different electronic devices.

Essentially, the logic of the application must store, for a certain topic, constructs (e.g. current and desired states from an user, that may represent a personal construct and the opposed personal construct respectively), elements (e.g. activities to reach a particular state), and ratings (e.g. feedback from the user about the recommended activity).

Possible future exploitation or use-cases based on the proposed solution for repertory grids acquisition may allow to model individuals decision making as an interacting agent-based system. Here the agent may be defined as the dynamical weighted network (based on repertory grids) that values the associations between elements and constructs. With this network of associations at a given space/time, it is possible to estimate the individual value function regarding the elements/constructs, helping in this way to predict the more probable outcome of a possible decision, by the agent.

Now, as an example, it is going to be described in detail a use-case in which a mobile application (in this case REM!X application, also known as REMIX) is going to be used in an embodiment of the present invention. It will be described the application logic (way of working) and model for extracting the repertory grids and the recommender system. This is only a non-limitative example and, as previously explained, many other electronic devices applications can be used for the solution proposed by the present invention.

Application Logic:

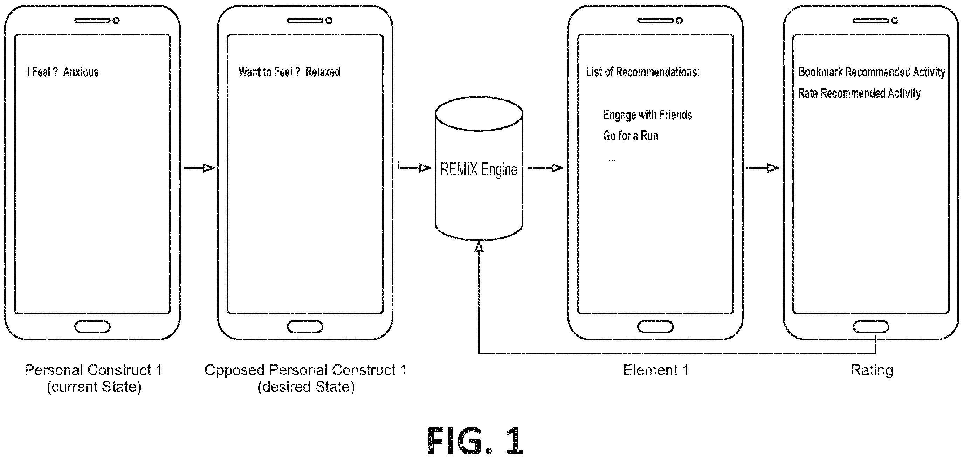

Figure 1 shows a schematic example on how a mobile application (REMIX) can be used for repertory grids extraction or, in other words, of the Logic Flow to digital repertory grids in the case of REEMIX App. Namely, the user is asked at time *t* to provide the information about "how he/she feels" (Personal construct 1, current state) and "how he/she wants to feel" (Opposed personal construct 1, desired state). The goal of the app is to provide the best match activity as a recommendation from a set of possible activities that will help the user achieve the goal "how he/she wants to feel". In order to do that, the REMIXs engine recommends a list of activities (Element 1) which are rated by the user.

For example, the user in the past acquired an "item A1" at time *t,* and an "item A2" at time *t+1* and the goal of a recommender system is based on this past information to prime the user to an item A3 or e.g. a ranked set of items {A,B,C,D, ...}. In the case of REMIX the analogy works as "how he/she feels" = "item A1" and "how he/she wants to feel" = "item A2". Actually, in the case of REMIX, the data acquired is a temporal list of pairs "how he/she feels" and "how he/she wants to feel" for times t1, t2, ..., tn. From this information, in an embodiment, it is built (through a data engine) a graphical model for each user. For example, a graph is built where the nodes are the different components of the repertory grids and the graph edges (lines) are the different unions/relations (preferably weighted) between said components; this graph will evolve in time with the user-app engagement. The model will be at will be the baseline or input of the recommender system.

That is, the user's information extracted from the electronic device application at each time is interpreted as different components of the repertory grids of the user at different times. A model/personal graph (which evolve in time) is built with the components of the extracted repertory grid for the user and their inter-relations (the model building will be explained in the following section). And said model/personal graph is feed to the recommender system to get the appropriated recommendations.

Model:

Now, it will be explained an example of how the model is built (this is a non-limitative example, and other ways of building the model can be used).

As explained above, from each user it is obtained (from the user interactions with the electronic device application) a temporal ordered list of pairs of personal construct and opposed personal construct (LU=[t, "how he/she feels", "how he/she wants to feel"], being t the time to which this information corresponds). This represents a bi-partite graph BG=[{X,Y},E{x,y}], where X and Y may be the set of node labels in the set of possible constructs X and opposed constructs Y ("how he/she feels" X and "how he/she wants to feel' Y') respectively, and E{x,y} the set of weighted edges that connects node x with y. In an embodiment, the weights are given by the frequency observed in the LU list (the frequency that X->Y) for each user. With the user engagement (user interactions with the electronic device through the application) LU increases and BG evolves over time.

The following text presents a real case labels for the REMIX app example.

```
array(['bored', 'stressed', 'annoyed', 'worried', 'angry', 'anxious',
       'relaxed', 'sleepy', 'sad', 'excited', 'miserable', 'content',
       'calm', 'happy', 'andventerous', 'creative', 'inspired', 'ashamed',
       'excited', 'calm', 'happy', 'productive', 'inspired', 'sleepy',
       'creative', 'content', 'relaxed', 'andventerous'], dtype=object)
```

From the BG per user, it is built its extended graph based on adjacency matrix and projections of the two states "how he/she feels" and "how he/she wants to feel". This extended graph represents the graphical model for each user.

For clarity purposes, it will be explained in detail how the extended graph is built, according to an embodiment of the invention, in the following non-limitative example.

1. The following table represents an example of data collected from the electronic device application (REMIX or other) in a period of time (for example, a week or any other period of time):

**Table 1**

| Observation order in time | I feel (variable X) | I want to feel (Y variable) |
|---|---|---|
| 1 | bored | productive |
| 2 | sleepy | excited |
| 3 | bored | productive |
| 4 | worried | calm |
| 5 | bored | productive |
| 6 | annoyed | calm |
| 7 | sad | happy |
| 8 | worried | happy |
| 9 | worried | calm |
| 10 | bored | excited |
| 11 | sleepy | excited |
| 12 | sad | happy |
| 13 | worried | happy |

(continued)

| Observation order in time | I feel (variable X) | I want to feel (Y variable) |
|---|---|---|
| ... | ... | ... |

2. From table 1, it is built a bipartite graph where nodes are variables (X,Y) and the edges are the frequency that X->Y at a specific time t. In table 2 we represent the adjacency matrix of the Bipartite Graph BG with data until time t=13, on variable (X,Y). Entries of the matrix represent the number of times that X->Y according to table 1.

**Table 2**

| X/Y | Productive | Excited | Calm | Happy |
|---|---|---|---|---|
| Bored | 3 | 1 | 0 | 0 |
| Sleepy | 0 | 2 | 0 | 0 |
| Worried | 0 | 0 | 2 | 2 |
| Annoyed | 0 | 0 | 1 | 0 |
| Sad | 0 | 0 | 0 | 2 |

3. From Table 2 (BG), the projections of XY into XX and YY are built. For these projections any similarity measure can be employed. In an embodiment, the Fuzzy Jaccard Similarity measure with (max,min) is used, since it has the property of creating semi-metric space, defined as ($v_{i,k}$ means the element k of vector i):

$$J_{(i,j)} = \frac{\sum_{k=1}^{n} \min(v_{i,k}, v_{j,k})}{\sum_{k=1}^{n} \max(v_{i,k}, v_{j,k})} \quad \text{(Equation 1)}$$

In the above specific example, for the XY into the XX projection the following vectors are considered (rows of table 2):

$X_1 = (3,1,0,0)$
$X_2 = (0,2,0,0)$
$X_3 = (0,0,2,2)$
$X_4 = (0,0,1,0)$
$X_5 = (0,0,0,2)$

Then to obtain the projection on XX, the fuzzy jaccard measure of all possible pairs $J(X_i, X_j) \equiv J(X_{i,j})$ is calculated according to eq.1;

$J(X_{1,1})=$ [min(3,3)+min(1,1)+min(0,0)+min(0,0)]/ [max(3,3)+max(1,1)+max(0,0)+max(0,0)]=

= (3+1+0+0)/(3+1+0+0)=4/4=1.0

$J(X_{1,2})=$ [min(3,0)+min(1,2)+min(0,0)+min(0,0)]/ [max(3,0)+max(1,2)+max(0,0)+max(0,0)]==

(0+1+0+0)/(3+2+0+0)=1/5=0.2

…

$J(X_{2,4})=$ [min(0,0)+min(2,0)+min(0,0)+min(0,2)]/ [max(0,0)+max(2,0)+max(0,0)+max(0,2)]=

= (0+0+0+0)/(0+2+0+2)=0/4=0.0

…

$J(X_{5,5})=$ [min(0,0)+min(0,0)+min(0,0)+min(2,2)]/ [max(0,0)+max(0,0)+max(0,0)+max(2,2)]=

= (0+0+0+2)/(0+0+0+2)=2/2=1

Then, the final result for t=13 of projection XX (Adjacency Matrix of the graph projection XX) is:

**Table 3**

| XX | Bored | Sleepy | Worried | Annoyed | Sad |
|---|---|---|---|---|---|
| Bored | 1.0 | 0.2 | 0.0 | 0.0 | 0.0 |
| Sleepy | 0.2 | 1.0 | 0.0 | 0.0 | 0.0 |
| Worried | 0.0 | 0.0 | 1.0 | 0.25 | 0.5 |
| Annoyed | 0.0 | 0.0 | 0.25 | 1.0 | 0.0 |
| Sad | 0.0 | 0.0 | 0.5 | 0.0 | 1.0 |

Continuing with the specific example of tables 1 and 2, for the XY into the YY projection, the following vectors are considered:

$Y_1=(3,0,0,0,0)$
$Y_2=(1,2,0,0,0)$
$Y_3=(0,0,2,1,0)$
$Y_4=(0,0,2,0,2)$

Therefore, according to eq.1;

$J(Y_{1,1})$= [min(3,3)+min(0,0)+min(0,0)+min(0,0)+min(0,0)]/

[max(3,3)+max(0,0)+max(0,0)+max(0,0)+max(0,0)]== (3+0+0+0+0)/(3+0+0+0+0)=3/3=1.0

$J(Y_{1,2})$= [min(3,1)+min(0,2)+min(0,0)+min(0,0)+min(0,0)]/

[max(3,1)+max(0,2)+max(0,0)+max(0,0)+max(0,0)]== (1+0+0+0+0)/(3+2+0+0+0)=1/5=0.2

…

$J(Y_{2,4})$= [min(1,0)+min(2,0)+min(0,2)+min(0,0)+min(0,2)]/

[max(1,0)+max(2,0)+max(0,2)+max(0,0)+max(0,2)]= (0+0+0+0+0)/(1+2+2+0+2)=0/7=0.0

…

$J(Y_{4,4})$= [min(0,0)+min(0,0)+min(2,2)+min(0,0)+min(2,2)]/

[max(0,0)+max(0,0)+max(2,2)+max(0,0)+max(2,2)]= (0+0+2+0+2)/(0+0+2+0+2)=4/4=

The final result for t=13 of projection YY (Adjacency Matrix of the graph projection YY) is:

**Table 4**

| YY | Productive | Excited | Calm | Happy |
|---|---|---|---|---|
| Productive | 1.0 | 0.2 | 0.0 | 0.0 |
| Excited | 0.2 | 1.0 | 0.0 | 0.0 |
| Calm | 0.0 | 0.0 | 1.0 | 0.4 |
| Happy | 0.0 | 0.0 | 0.4 | 1.0 |

4. In an embodiment, scale effects between XY, XX and YY are removed. This is done, because, in general, XY, XX and YY are in different scales, and the scale effects should be removed or at least reduced. In order to reduce the scale effects, a z-score may be used. Here, XY is z-scored to obtain the matrix ZXY considering for the calculation only the non-zero elements of XY, i.e. for the z-score calculation all zeros from table 2 are excluded. Similarly, it is done on XX and YY, where the z-score calculation is based on tables 3 and 4, removing from the calculation the non-zero values and as well as the diagonal elements in this case, respectively. It is obtained the matrixes ZXY, ZXX and ZYY.

**Table 5**. ZXY adjacency matrix.

| ZXY | Productive | Excited | Calm | Happy |
|---|---|---|---|---|
| Bored | 1.6562 | -1.2421 | 0 | 0 |
| Sleepy | 0 | 0.2070 | 0 | 0 |
| Worried | 0 | 0 | 0.2070 | 0.2070 |
| Annoyed | 0 | 0 | -1.2421 | 0 |
| Sad | 0 | 0 | 0 | 0.2070 |

**Table 6**. ZXX adjacency matrix.

| ZXX | Bored | Sleepy | Worried | Annoyed | Sad |
|---|---|---|---|---|---|
| Bored | 0.0 | -0.8115 | 0.0 | 0.0 | 0.0 |
| Sleepy | -0.8115 | 0.0 | 0.0 | 0.0 | 0.0 |
| Worried | 0.0 | 0.0 | 0.0 | -0.4637 | 1.2753 |
| Annoyed | 0.0 | 0.0 | -0.4637 | 0.0 | 0.0 |
| Sad | 0.0 | 0.0 | 1.2753 | 0.0 | 0.0 |

**Table 7.** ZXX adjacency matrix.

| ZYY | Productive | Excited | Calm | Happy |
|---|---|---|---|---|
| Productive | 0.0 | -0.8660 | 0.0 | 0.0 |
| Excited | -0.8660 | 0.0 | 0.0 | 0.0 |
| Calm | 0.0 | 0.0 | 0.0 | 0.8660 |
| Happy | 0.0 | 0.0 | 0.8660 | 0.0 |

From these adjacency matrixes we build the z-scored Extended graph of XY:

$$ZE_{BG} = \begin{bmatrix} ZXX & ZXY \\ ZXY^T & ZYY \end{bmatrix}$$

5. Normalized Extend graph: Next, in an embodiment, it is proceeded into a normalization of z-scored $ZE_{BG}$, for example, removing for this calculation all non-zero entries and diagonal elements, and normalize it into the unit interval [0,1] by means of a unique linear function that maps W:[a,b] ->[0,1] (equation 2).

$$w_{ij} = \frac{(1-2\varepsilon)Z_{ij}+(2\varepsilon-1)\min(Z)}{\max(Z)-\min(Z)} + \varepsilon \text{ (equation 2)}$$

With this process, it is obtained the scaled and normalized graphs $E_{BG}$, with e.g. $\varepsilon$ = 0.01. The extended graph in this case is (that is, in an embodiment, the extended graph will be built with the nodes and weights of the following table):

**Table 8.** Normalized extended graph $E_{BG}$.

| $E_{BG}$ | Bored | Sleepy | Worried | Annoyed | Sad | Productive | Excited | Calm | Happy |
|---|---|---|---|---|---|---|---|---|---|
| Bored | 0 | 0.1556 | 0 | 0 | 0 | 0.9900 | 0.0100 | 0 | 0 |
| Sleepy | 0.1556 | 0 | 0 | 0 | 0 | 0 | 0.5000 | 0 | 0 |

(continued)

| $E_{BG}$ | Bored | Sleepy | Worried | Annoyed | Sad | Productive | Excited | Calm | Happy |
|---|---|---|---|---|---|---|---|---|---|
| Worried | 0 | 0 | 0 | 0.2732 | 0.8612 | 0 | 0 | 0.5000 | 0.5000 |
| Annoyed | 0 | 0 | 0.2732 | 0 | 0 | 0 | 0 | 0.0100 | 0 |
| Sad | 0 | 0 | 0.8612 | 0 | 0 | 0 | 0 | 0 | 0.5000 |
| Productive | 0.9900 | 0 | 0 | 0 | 0 | 0 | 0.1372 | 0 | 0 |
| Excited | 0.0100 | 0.5000 | 0 | 0 | 0 | 0.1372 | 0 | 0 | 0 |
| Calm | 0 | 0 | 0.5000 | 0.0100 | 0 | 0 | 0 | 0 | 0.7228 |
| Happy | 0 | 0 | 0.5000 | 0 | 0.5000 | 0 | 0 | 0.7228 | 0 |

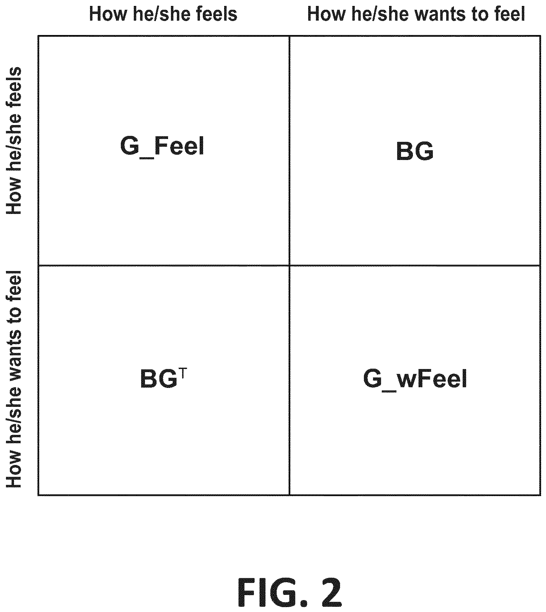

Figure 2 shows a schematic example of the extended graph from the bi-partite graph BG, according to an embodiment of the present invention. BG is the original bi-partite graph (built as previously explained), G_Feel is a projection of the BG to the personal construct nodes X, current state ("How he/she feels"), G_wFeel is a projection of the BG to the opposed personal construct nodes Y, desired state ("How he/she wants to feel") and $BG^T$ is the transpose of the BG. In all four corners the scale effect may be removed/reduced (for example, as explained in Simas Tiago, Chavez Mario, Rodriguez Pablo, Diaz-Guilera Albert, (2015). "An algebraic topological method for multimodal brain networks comparisons" Frontiers in Psychology, 6, p. 904. DOI=10.3389/fpsyg.2015.00904) as it has been previously explained.

Summarizing, the information observed from the user is the bi-partite graph BG as described above. G_Feel and G_wFeel are obtained by projecting BG information on X and Y, respectively. These projections may be built using similarity measures such as Jaccard similarity measure, cosine, etc. In general these 4 corners of the extended graph are embedded in different scales and, the scale effects can be removed using different known procedures. The final extended graph is obtained after the projections, removal of scale effects (optional) and normalization (optional), for, example with values in the unit interval [0,1]. It is worth noting that with the engagement of the user through the application, BG evolves in time and therefore its extended graph representation. The extended graph is a graphical model of the user that, in an embodiment, it is used as a building block for a recommender system.

Recommender system:

According to an embodiment of the invention, after having the model described above and the current reported state at time $t$, that is, [$t$, "how he/she feels", "how he/she wants to feel"] for the case of the REMIX application], the state vector of the model is built. The state vector of the model consists of a vector with the order labels XY and filled with zeros in all entries except with "1" in the "How he/she want to feel" reported. This vector sets the values of the diagonal matrix representation of the graphical model (figure 2).

Given the model and state vector, in an embodiment, the recommender system may calculate the n-diffusion closure distance (for example, as proposed in the document SIMAS, T., & ROCHA, L. (2015). Distance closures on complex networks. Network Science, 3(2), 227-268. doi:10.1017/nws.2015.11), as follows:

Let $E_{BG}$ the normalized extended graph, where weights represent some kind of similarity. That is, weights close to 1.0 are very similar and close to 0.0 not similar. Convert $E_{BG}$ into $DE_{BG}$, converting all weights entries in $E_{BG}$, $w_{ij}$ into $d_{ij}$ with $d_{ij} =$

$$\varphi\left(w_{ij}\right) = \frac{1}{w_{ij}} - 1.$$

Consider the dot product of two vectors defined by:

$$v_i \cdot v_j = \sum_k^{\oplus} v_{ik} \otimes v_{jk}$$

Where:

$$x \oplus y = \begin{cases} \frac{xy}{x+y}, x, y \in ]0,1[ \\ y \text{ or } x, \text{if } x = +\infty \text{ or } y = +\infty \\ 0, \text{if } x = 0 \text{ or } y = 0 \end{cases}, \quad \forall x, y \in [0, +\infty]$$

$$x \otimes y = x + y \ , \ \forall x, y \in [0, +\infty]$$

The n-diffusion closure distance of $E_{BG}$ is then the n-dot product of $E_{BG}$ with it-self n-times:

$$DE_{BG}{}^2 = DE_{BG} \cdot DE_{BG}$$

$$DE_{BG}{}^n = DE_{BG} \cdot DE_{BG}{}^{n-1}$$

The value of n is usually selected as the value of the diameter of the extended graph. It can be used as an estimate the average shortest path length, which analytical formula is n~log(#nodes) for a random graph.

This process represents the state vector sets, i.e. the set of sources and sinks in the diffusion process of the extended graph. The sink can be seen as the goal or reward to be obtained, and the sources all possible initial positions. The n-diffusion closure distance sets a diffusion path between any source to the sink. The reported "How he/she feels" sets a position in the path. The distance between "How he/she feels" and "how he/she wants to feel" gives a score on how achievable is the sink/reward from the initial position as well as a set of policies to follow in order to reach the reward. Below, it is given a concrete example.

For clarity purposes, a non-limitative example will be explained now:

Suppose the model (as in figure 2) extended graph for a specific user, and at a given time *t*, he/she reports: [t, "Bored", "Productive"], that is at *t* he/she feels "bored" and wants to feel "productive".

Now, suppose the set of X nodes are: {bored, stressed, anxious, sleepy, annoyed} and the set of Y nodes are: {calm, happy, productive, content, relaxed, excited}. In this hypothetical example the state vector or diagonal of the model is labelled as:

*State_vector=[bored, stressed, anxious, sleepy, annoyed, calm, happy, productive, content, relaxed, excited]*

Therefore, the state_vector is set with all zeros except for the entry "productive" (How he/she wants to feel, as reported at given time *t*,):

1. Normalization factor (all zeros): norm_vector=[0,0,0,0,0,0,0,0,0,0,0] and apply the n-diffusion, as described above, and store the norm_memory (diagonal of the resulting n-diffusion), which corresponds to a scenario where there is no sinks and therefore the diffusion process represents a random walker in this graphical environment;

2. Set a state_vector=[0,0,0,0,0,0,0,1,0,0,0];

3. Set the diagonal of the user model with the state vector;

4. Calculate the distance n-diffusion;

5. Extract the diagonal vector (memory) from the distance n-diffusion, then divide pointwise the vector with the norm_vector (memory), and sort the results in ascending order.

6. At the top appears "productive" the set sink or reward followed by the other labels of the state vector.

Below, table 9, shows the n-diffusion ordered vector obtained from a REM!X user when he/she wants to be productive (First column labelled vector, and the second column diffusion distance to the sink "Productive"). The order represents the diffusion shortest path between any source to the sink ("Productive"). In table 9, the meaning of (-),(+) is: (-) current feeling (I feel) 'X' and (+) the target feeling (want to feel) 'Y', respectively. That is, happy (+) means that the user comes from a 'X' feeling (I feel) and wants to feel happy 'Y', and happy (-) means that the user comes from 'X' feeling happy (I feel happy) and wants to change this feeling to another (want to feel) 'Y'.

**Table 9. Final state vector after distance n-diffusion.**

| | |
|---|---|
| **Productive(+)** | 0 |
| *adventurous( +)* | 0.9796 |

(continued)

| | |
|---|---|
| *relaxed(+)* | 0.9847 |
| *bored(-)* | 0.9955 |
| *content(+)* | 0.9956 |
| *stressed(-)* | 0.9985 |
| *excited(+)* | 0.99865 |
| *inspired(+)* | 0.99867 |
| *calm(+)* | 0.99884 |
| *creative(+)* | 0.99885 |
| *happy(+)* | 0.99899 |
| calm(-) | ... |
| content(-) | ... |
| miserable(-) | ... |
| annoyed(-) | ... |
| inspired(-) | ... |
| sleepy(-) | ... |
| relaxed(-) | ... |
| anxious(-) | 0.99999851 |
| sleepy(+) | Not accessible |
| angry(-) | Not accessible |
| creative(-) | Not accessible |
| worried(-) | Not accessible |
| adventurous(-) | Not accessible |
| excited (-) | Not accessible |
| sad(-) | Not accessible |
| ashamed(-) | Not accessible |
| happy(-) | Not accessible |

Diffusion distance values higher than a certain threshold (in table 9 case, as a non-limitative example, this threshold is 0.999), are considered random or, in other words, said nodes/feelings whose diffusion distance value is lower than said threshold (in bold letters) are not considered random. The nodes/feelings whose diffusion distance value is higher that the threshold are considered trapped (random) as they hardly have access to the desired feeling, in this case Productive (+).

In this case, it is observed that between "bored(-)" and "productive(+)" has "relaxed(+)" and "adventurous(+)" in the middle, which means when the user is "bored(-)" tries to be "relaxed(+)" and "adventurous(+)" towards being "productive". From this diffusion path (table 9), it can be seen that the best chance that the user gets "productive" is from "happy(+)": happy (+) as explained above, is the last state that may reach "Productive (+)" (the target), based on the threshold; all the other ones below "happy (+)" hardly reach "Productive (+)", since the diffusion process from those is trapped. i.e. the diffusion from those other do not reach (no access to) the set sink "Productive (+)", or its diffusion into the sink is very low. Moreover, the constructs in-between, "happy(+)" and "productive(+)", gives the information of the policies to follow. That is, what type of recommendations in this case activities should we recommend, which are the ones between "happy(+)" and "productive(+).

Note that for this particular user it would be particularly very difficult to achieve "productive" if the user is in one of the constructs on the tail "sleepy(-)" to "happy(-)", which resonates with common intuition. That is, after obtaining from the model and initial state the diffusion path, the recommender system will recommend a scored list of activities associated with constructs between "Productive (+)" and "happy(+)". That is, in this specific case (table 9), it is known that the user is X='bored (-)', which is in between the reachable list between 'Happy (+)' and Y='Productive (+)'; in between these two (X='bored (-)' and Y='Productive (+)') 'relaxed (+)' and adventurous (+)' appear; therefore it will be recommended activities that first prime the user into 'Relaxed (+)' followed by other activities that prime the user into Adventurous (+)' followed to Y='Productive'.

In summary, from mobile apps (such as REMIX), it is built a personalized graphical model, then based on the reported or e.g. the identification of an initial state based on the variables X, Y (see above), n-diffusion closure distance is calculated after setting the target goals (sinks in a diffusion process) determining the shortest diffusion path between the possible sources and sinks. Given the initial state, it can be determined in which position the user is in this path and the policies that are in between. With this information, the recommender system acts as selecting, for example, the best activities that match the policies that are in between the initial state and the target goal.

Depending on the embodiment, the modules or electronic means/processors (applying the different explained processes) are in the same or in different physical places (different devices). In an embodiment, all modules are performed in the same device (for example, a central or remote server or an electronic device of the user). In other embodiments, the modules (except the mobile communications device itself) are residing on one or more servers. In other embodiments, some (or all) modules may be in the user's electronic device and the rest, if any, in one or more servers. In the case that the modules are in a server, the user electronic device communicates with the server through a communication networks (a wireless communications network, a mobile communications network as 2G, 3G, 4G or LTE, LAN or W-LAN or any other type of communication networks...).

The description and drawings merely illustrate the principles of the invention.

Although the present invention has been described with reference to specific embodiments, it should be understood by those skilled in the art that the foregoing and various other changes, omissions and additions in the form and detail thereof may be made therein without departing from the scope of the invention as defined by the following claims. Furthermore, all examples recited herein are principally intended expressly to be only for pedagogical purposes to aid the reader in understanding the principles of the invention and the concepts contributed by the inventor(s) to furthering the art, and are to be construed as being without limitation to such specifically recited examples and conditions. Moreover, all statements herein reciting principles, aspects, and embodiments of the invention, as well as specific examples thereof, are intended to encompass equivalents thereof.

It should be appreciated by those skilled in the art that any block diagrams herein represent conceptual views of illustrative circuitry embodying the principles of the invention. Similarly, it will be appreciated that any flow charts, flow diagrams, state transition diagrams, pseudo code, and the like represent various processes which may be substantially represented in computer readable medium and so executed by a computer or processor, whether or not such computer or processor is explicitly shown.

**Claims**

1. A computer implemented method for efficient extraction of personal users' data and improvement of the wellbeing state of users, the method comprising the following step performed by one or more electronic processors of an electronic system:

   a) Receiving and storing in a database, data related to one or more users, where data related to a user is acquired through interactions of the user with an application running in an electronic device of said user;
   b) Generating a graphical model for at least one user by processing a set of data related to said user, obtained from the database, corresponding to a certain period of time; where the nodes of the graphical model are the different values of the set of data;
   c) For each user for which a graphical model has been generated, determining a recommended range of values for parameters or activities related to the user based at least on the generated graphical model for the user, using state vectors and diffusion metrics obtained from the graphical model generated for the user.

2. The method according to claim 1, where the database is a database located in an electronic device of a user or the database is a database external to the electronic devices of the one or more users, stored in a server, and the electronic devices of the users send the acquired data to the server, through a wire or wireless communications network.

3. The method according to any of the preceding claims, where the electronic devices of the user are one of the following: a laptop, a tablet, a personal computer, a portable computer, a mobile phone, a smartphone or a smartwatch.

4. The method according to any of the preceding claims where step b) further includes updating the graphical model based on new set of data, acquired through the application running in the electronic device of the user, corresponding to a different later period of time; and/or updating the generated graphical model for an user every time new data for said user, acquired through user interactions with the application running in the user's electronic device, is received and stored after the certain period of time.

5. The method according to any of the preceding claims, where the set of data related to a user is a temporal ordered list of pairs of values of personal construct, X, and opposed personal construct, Y, of the user corresponding to the certain period of time and the graphical model for an user includes a graph, Bipartite Graph, based on the frequency that a specific pair of values of personal constructs and opposite personal construct (X,Y) appear in the list.

6. The method according to claim 5, where the graphical model for an user comprises an extended graph which includes the Bipartite Graph, the projection of the Bipartite Graph to the personal construct nodes X and the projection of the Bipartite Graph to the opposed personal construct nodes Y, where said projections are built using a similarity measure.

7. The method according to claim 6, where the similarity measure used is the Fuzzy Jaccard Similarity measure.

8. The method according to any of the claims 6-7. where said extended graph also includes the transpose of the Bipartite Graph.

9. The method according to any of the claims 6-8, where to build said extended graph, the scale effect between the Bipartite Graph and the projections is reduced.

10. The method according to claim 9 where, in order to reduce the scale effect a z-score technique is used.

11. The method according to any of the claims 5-10, where the personal construct and the opposed personal construct is a current state and a desired state respectively of the user.

12. The method according to claim 1, where step c) comprises:

   c1) obtaining and initial state and a target wellbeing goal for a user;
   c2) based on the graphical model generated for said user and on the initial state and the target goal, apply n-diffusion closure distance to determine the shortest diffusion path between the initial state ant the target goal;
   c3) based on said determined shortest diffusion path, determining a recommended range of values for parameters or activities related to the user for achieving the target goal from the initial state of the user.

13. The method according to any of the preceding claims, where the determined recommendation for a user is presented to the user through a user interface of the electronic device of the user.

14. An electronic system for efficient extraction personal users' data and improvement of the wellbeing state of users, the system comprising:

   - Means for receiving through a communications network and storing in a database, data related to one or more users, where data related to a user is acquired through interactions of the user with an application running in an electronic device of said user;
   - at least one processor configured to:

      - Obtain from the database a set of data related to at least one user corresponding to a certain period of time;
      - Generate a graphical model for the at least one user by processing the set of data of related to said user corresponding to the certain period of time; where the nodes of the graphical model are the different values of the set of data;
      - For each user for which a graphical model has been generated, determining a recommended range of values for parameters or activities related to the user based at least on the generated graphical model for the user, using state vectors and diffusion metrics obtained from the graphical model generated for the user.

15. A non-transitory computer readable medium, comprising instructions for causing a computer device to perform the method of any of the claims 1-13.

I Feel ? Anxious

Want to Feel ? Relaxed

REMIX Engine

List of Recommendations:

Engage with Friends
Go for a Run
...

Bookmark Recommended Activity

Rate Recommended Activity

Personal Construct 1
(current State)

Opposed Personal Construct 1
(desired State)

Element 1

Rating

# FIG. 1

EP 3 916 603 A1

|  | How he/she feels | How he/she wants to feel |
|---|---|---|
| How he/she feels | G_Feel | BG |
| How he/she wants to feel | $BG^{\top}$ | G_wFeel |

# FIG. 2

Europäisches
Patentamt

European
Patent Office

Office européen
des brevets

# EUROPEAN SEARCH REPORT

**Application Number**

EP 20 38 2454

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/032670 A1 (BRAZELL ROBERT [US]) 29 January 2015 (2015-01-29) * paragraphs [0030] - [0036] * ----- | 1-15 | INV. G06F21/62 G06Q30/02 |
| Y | US 2018/218289 A1 (ALBRECHT HENRY [US]) 2 August 2018 (2018-08-02) * abstract; figures 1,6,7,10,13,16c * ----- | 1-15 | |
| Y | US 2017/277746 A1 (LEE SEOK WON [KR] ET AL) 28 September 2017 (2017-09-28) * paragraphs [0042] - [0046]; figure 5 * ----- | 1-15 | |

| TECHNICAL FIELDS SEARCHED (IPC) |
|---|
| G06F G06Q |

The present search report has been drawn up for all claims

| Place of search | Date of completion of the search | Examiner |
|---|---|---|
| Munich | 27 October 2020 | Kerschbaumer, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 38 2454

This annex lists the patent family members relating to the patent documents cited in the above-mentioned European search report.
The members are as contained in the European Patent Office EDP file on
The European Patent Office is in no way liable for these particulars which are merely given for the purpose of information.

27-10-2020

| Patent document cited in search report | Publication date | Patent family member(s) | Publication date |
|---|---|---|---|
| US 2015032670 A1 | 29-01-2015 | NONE | |
| US 2018218289 A1 | 02-08-2018 | US 2008109257 A1<br>US 2009319347 A1<br>US 2018218289 A1<br>WO 2008008514 A2 | 08-05-2008<br>24-12-2009<br>02-08-2018<br>17-01-2008 |
| US 2017277746 A1 | 28-09-2017 | KR 101756196 B1<br>US 2017277746 A1 | 10-07-2017<br>28-09-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FACCIO, E. ; CASTIGLIONI, M. ; BELL, R. C.** Extracting information from repertory grid data: New perspectives on clinical and assessment practice. *TPM - Testing, Psychometrics, Methodology in Applied Psychology,* 2012, vol. 19 (3), 177-196

- **SIMAS TIAGO ; CHAVEZ MARIO ; RODRIGUEZ PABLO ; DIAZ-GUILERA ALBERT.** An algebraic topological method for multimodal brain networks comparisons. *Frontiers in Psychology,* 2015, vol. 6, 904
- **SIMAS, T. ; ROCHA, L.** Distance closures on complex networks. *Network Science,* 2015, vol. 3 (2), 227-268